# EUROPEAN PATENT APPLICATION

(11) **EP 3 925 702 A1**
(43) Date of publication of application: **22.12.2021**
(21) Application number: 20180032.3
(22) Date of filing: 15.06.2020
(51) Int. Cl.: B01L 3/00, C12M 1/34, C40B 50/08, C40B 60/14, C12Q 1/02, C12M 1/12, C12M 1/00, G01N 15/14

(54) **METHOD AND SYSTEM OF PRODUCING A LIBRARY OF MICROORGANISMS**

(71) Applicant: Biomillenia SAS, 93230 Romainville (FR)
(72) Inventor: Du, Guansheng, 94200 Ivry sur Seine (FR); Thomas, Laurine, 56480 Cléguérec (FR); Dajkovic, Aleksander, 75018 Paris (FR); Loeffert, Dirk, 42781 Haan (DE)
(74) Representative: CH Kilger Anwaltspartnerschaft mbB

(57) **Abstract**

In a first aspect, the present invention relates to a method of producing a library of microorganisms, the method comprising the steps of:
a. providing a first fluid comprising at least one single cell,
b. dispersing said first fluid comprising at least one single cell in a second fluid, thereby obtaining a plurality of single-layer microfluidic droplets, wherein at least one single-layer microfluidic droplet comprises at least one single cell, wherein the second fluid is immiscible with the first fluid,
c. optionally, adding to said at least one single-layer microfluidic droplet a third fluid comprising a sensing compound, wherein the third fluid is miscible with said first fluid, and wherein the third fluid is immiscible with said second fluid,
d. injecting said at least one single-layer microfluidic droplet optionally comprising the sensing compound into a fourth fluid, wherein said fourth fluid is immiscible with said second fluid, thereby obtaining at least one double-layer microfluidic droplet,
e. dispensing said at least one double-layer microfluidic droplet into a culture medium based on the viability of the cell,
f. incubating said culture medium , thereby obtaining said library.

In a second aspect, the present invention relates to a system comprising:
a. a first microfluidic chip for producing a plurality of single-layer microfluidic droplets wherein at least one single-layer microfluidic droplet comprisesat least one single cell,
b. a first microfluidic device for collecting said plurality of single-layer microfluidic droplets,
c. a second device for adding a sensing compound into said at least one single-layer microfluidic droplet comprising at least one single cell,
d. a second microfluidic chip for producing a double-layer microfluidic droplet, and
e. a dispensing unit.

In a third aspect, the present invention relates to the use of the method according to the first aspect of the present invention in a system according to the second aspect of the present invention.

## Description

### Field of the invention

The present invention is in the field of microorganism strain culture and analysis by means of microfluidic devices. The present invention is also in the field of microfluidics, particularly in the field of microfluidic cell culture, analysis and sorting devices.

### Background of the invention

Microbial resources are essential to understand life sciences because microorganisms are crucial to maintain the external environment as well as the inner ecosystem of humans, higher animals and plants.

Identifying and understanding the microorganisms at the strain level has a great impact in the field of health, biotechnology, agriculture, food technology and for research in the life sciences. With reference to the health, for example, this is an important issue as some strains, even within the same species, may be pathogenic for humans while others are not. Therefore, the differentiation between these two groups, pathogenic and non-pathogenic, is crucial in clinical microbiology and in medical and pharmaceutical treatment. Strain level identification is important not only in the matter of determining whether a patient should be prescribed an antibiotic, but also in terms of selecting the appropriate drug (e.g., when a specific bacterial strain is resistant to a particular antibiotic).

In the last decade, microfluidic devices have been proposed as a potential platform for high-throughput screening technology because of their properties of low sample consumption, low analysis cost, easy handling of nanoliter-volumes of liquids and being suitable for cell-based assays. To date, different components for microfluidic based cell-based high throughput screening platforms have been developed, including cell culture, introduction and transportation of samples and characterization of cell viability (Du et al. 2016, Analytica Chimica Acta 903:36-50).

In order to investigate the strains of microorganisms, culture collections play a crucial role in their long-term and stable preservation.

The current methods of building microbial collections show severe limitations. On the one hand, they are very labor intensive and require the mobilization of significant laboratory resources. On the other hand, most current methods of building microbial collections suffer from limited diversity that they can access. This limited diversity of microbes that standard methods in microbiology are capable of capturing is well known in microbiology to represent a small part of the total microbial diversity. This is why the remaining microbial diversity is often called microbial dark matter. Finally, standard methods do not easily allow investigation of thousands of strains in parallel.

In view of the above limitations affecting the current methods, development of an improved method for the identification and collection of single strains would be beneficial.

### Summary of the invention

In a first aspect, the present invention relates to a method of producing a library of microorganisms, the method comprising the steps of:
a. providing a first fluid comprising at least one single cell,
b. dispersing said first fluid comprising at least one single cell in a second fluid, thereby obtaining a plurality of single-layer microfluidic droplets, wherein at least one single-layer microfluidic droplet comprises at least one single cell, wherein the second fluid is immiscible with the first fluid,
c. optionally, adding to said at least one single-layer microfluidic droplet a third fluid comprising a sensing compound, wherein said third fluid is miscible with said first fluid, and wherein said third fluid is immiscible with said second fluid,
d. injecting said at least one single-layer microfluidic droplet optionally comprising the sensing compound into a fourth fluid, wherein said fourth fluid is immiscible with said second fluid, thereby obtaining at least one double-layer microfluidic droplet,
e. dispensing said at least one double-layer microfluidic droplet into a culture medium based on the viability of the cell,
f. incubating said culture medium, thereby obtaining said library.

In a second aspect, the present invention relates to a system comprising:
a. a first microfluidic chip for producing a plurality of single-layer microfluidic droplets wherein at least one single-layer microfluidic droplet comprises at least one single cell,
b. a first microfluidic device for collecting said plurality of single-layer microfluidic droplets,
c. optionally, a second microfluidic device for adding a sensing compound into said at least one single-layer microfluidic droplet comprising at least one single cell,
d. a second microfluidic chip for producing a double-layer microfluidic droplet, and
e. a dispensing unit.

In a third aspect, the present invention relates to the use of the method according to the first aspect of the present invention in a system according to the second aspect of the present invention.

### Brief description of the figures

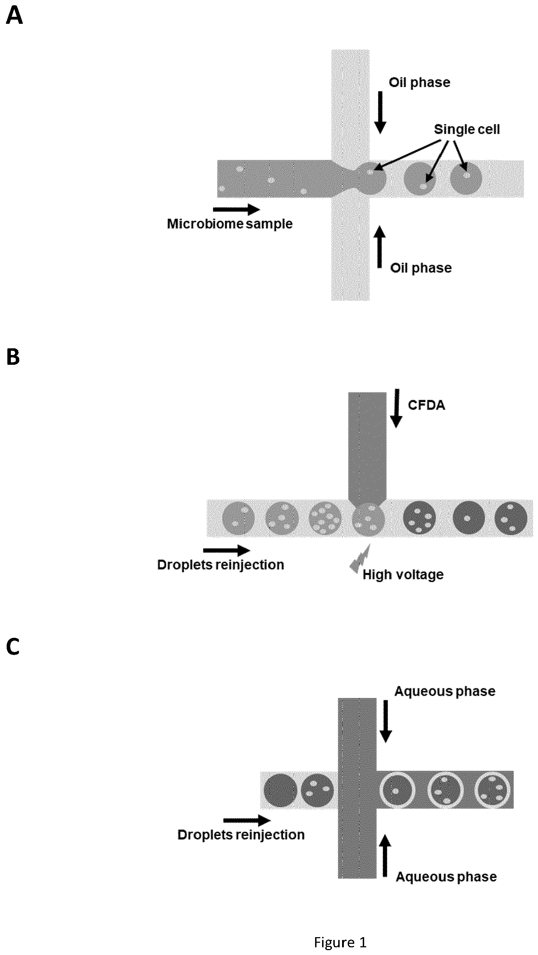

Figure 1 shows the method for producing a water-in-oil-in-water (W/O/W) microfluidic droplets.
These droplets are also referred to as single-layer microfluidic droplets.

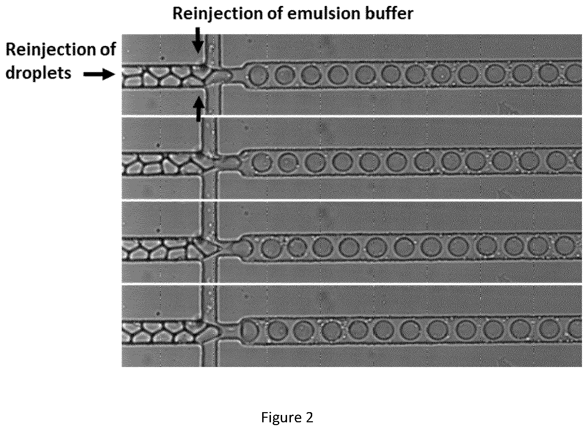

Figure 2 shows a frame of the process of making the water-in-oil-in-water (W/O/W) microfluidic droplets.

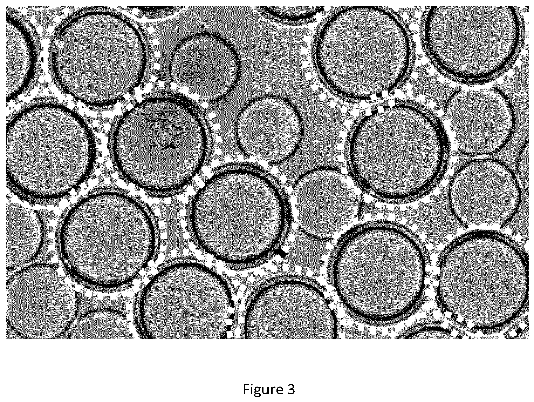

Figure 3 shows the presence of growth of microorganisms in water-in-oil-in-water (W/O/W) microfluidic droplets after incubation (see the droplets circled by the dotted line).

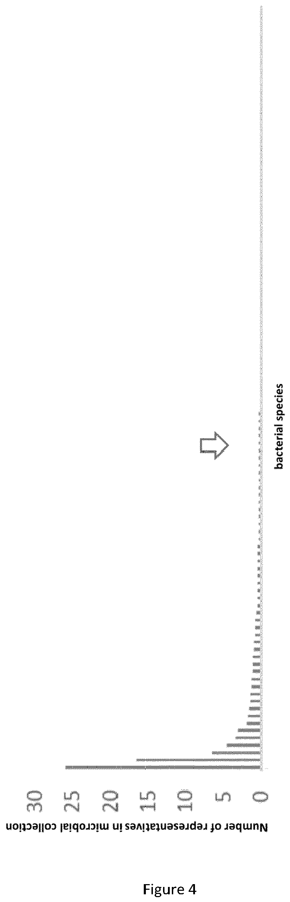

Figure 4 shows the distribution of abundance of 80000 strains in a culture collection obtained using the method described herein. More than 50% of the strains are present only once in the culture collection.

### Detailed description of the invention

The present invention is intended to solve the problem of isolation, analysis and collection of single strains within a multi-strain microorganism culture in a high-throughput fashion. In particular, the invention disclosed herein focuses on the production of a library of pure microorganisms, which among other things allow the characterization of complex microbial cultures at the strain level.

The following aspects and embodiments describe the claimed invention.

In a first aspect, the present invention relates to a method of producing a library of microorganisms, the method comprising the steps of:
a. providing a first fluid comprising at least one single cell,
b. dispersing said first fluid comprising at least one single cell in a second fluid, thereby obtaining a plurality of single-layer microfluidic droplets, wherein at least one single-layer microfluidic droplet comprises at least one single cell, wherein the second fluid is immiscible with the first fluid,
c. optionally, adding to said at least one single-layer microfluidic droplet a third fluid comprising a sensing compound, wherein said third fluid is miscible with said first fluid, and wherein said third fluid is immiscible with said second fluid,
d. injecting said at least one single-layer microfluidic droplet optionally comprising the sensing compound into a fourth fluid, wherein said fourth fluid is immiscible with said second fluid, thereby obtaining at least one double-layer microfluidic droplet,
e. dispensing said double-layer microfluidic droplet into a culture medium based on the viability of the cell,
f. incubating said culture medium, thereby obtaining said library.

In the context of the present invention, the term "library of microorganisms" refers to a collection of viable single strains that has been prepared from the selected cell clone under defined conditions, dispensed into multiple containers and stored under defined conditions. The living strains may be safely kept long term and the information regarding these strains may be individually identified, registered and managed. When associated to a storage facility, the term "library of microorganisms" also refers to a cell bank.

The microfluidic droplets may be produced by any suitable technique known in the art. The droplets may be spherical or non-spherical. In the latter case, the diameter of a droplet may be taken as the diameter of a perfect sphere having the same volume as the non-spherical droplet.

In the context of the present invention, the droplets may have an average volume of less than 1 µL, less than 500 nL, less than 10 nL, less than 1 nL, less than 100 pL, less than 50 pL, less than 30 pL or less than 15 pL.

The droplets are also contained in a microfluidic channel or microfluidic chip. The microfluidic channel has an average inner diameter of less than 1 mm, preferably less than 100 µm, more preferably less than 10 µm, even more preferably less than 1 µm.

In one embodiment, the fluid forming the droplets is substantially immiscible with the carrying fluid surrounding the droplets. For example, the fluid may be hydrophilic, while the carrying fluid may be hydrophobic or *vice versa.* Generally, a "hydrophilic fluid" is one that is miscible with pure water, while a "hydrophobic fluid" is a fluid that is immiscible with pure water.

In the context of the present invention, for example, the first fluid is hydrophilic and miscible with the third and fourth fluids and the second fluid is hydrophobic and immiscible with the first, third and fourth fluids.

The first fluid, third and fourth fluid may be a hydrophilic solution, e.g., an aqueous solution, and the second fluid may be a hydrophobic solution, e.g., an oil solution.

In one embodiment, the first fluid, the third fluid and the fourth fluid are hydrophilic and the second fluid is hydrophobic or *vice versa.*

As used herein, the term "single-layer microfluidic droplet" refers to a microfluidic droplet surrounded or enveloped by a layer of a fluid immiscible with the first fluid comprising the droplet. As used herein, the term "double-layer microfluidic droplet" refers to a single-layer microfluidic droplet surrounded or enveloped by a layer of a fluid immiscible with the second fluid surrounding or enveloping the droplet.

In another embodiment, the single-layer microfluidic droplet is a water-in-oil emulsion droplet or *vice versa.*

The encapsulation of the microorganisms into the droplets are well known in the state of the art. Encapsulation of the microorganisms into the droplets may be performed by any existing technique. Preferably, the microorganisms are encapsulated into the droplets by prior appropriate dilution of the microorganisms into the aqueous phase used for droplet creation. Alternatively, microorganisms may be encapsulated through injection of an aqueous volume into droplets or by droplet fusion.

In one embodiment, the single cell is obtained by diluting a microorganism sample comprising a plurality of strains. As used herein, the term "strains" refers to microorganisms of a particular species which have common characteristics. The term "strains" refer to a pure culture where strains are obtained from a single cell colony. In the context of the present invention, the terms "strain" and "single cell" may be used interchangeably.

In order to increase the fraction of microfluidic droplets comprising a single cell over the number of droplets comprising multiple cells, the inventors have found that a dilution of the original microorganism sample is beneficial.

In view of the dilution of the original microorganism sample and following a Poisson distribution, the majority of droplets results empty. In other cases, more than one single cell may be present in a droplet. Otherwise, a specialized microfluidic chip will be added to generate Dean-flow. In the Dean-flow, the cells are equally distributed, therefore, in this scenario, most of the droplets will contain only one single cell.

In one embodiment, it is preferred that a microfluidic droplet comprises at least one single cell, more preferably one single cell.

In another embodiment, no more than about 25%, no more than about 15%, no more than about 10% no more than about 5% or no more than about 1% of the microfluidic droplets contains more than one single cell therein.

In another embodiment, the microfluidic droplet comprises more than one single cell.

If a droplet comprises more than one single cell, the inventors have found that one strain may grow faster than another one and occupy the entire volume within the droplet. In some cases, the presence of more than one single cell may be beneficial for the growth of one strain. The result obtained, after dispensation of the droplet in the microtiter plate, is still a single cell colony.

The method disclosed herein is suitable for producing a library of microorganisms, wherein the microorganisms may be cultivated in an identical culture medium or in different culture media. Therefore, the present method allow to culture in parallel different microorganisms according to their ideal growth medium. On the other hand, the presence of a specific culture medium may allow the selection of a target strain.

In one embodiment, each of the plurality of single-layer microfluidic droplets comprise an identical culture medium.

In another embodiment, each of the plurality of single-layer microfluidic droplets comprise a unique culture medium.

The method disclosed herein may comprise additional incubation steps of the microfluidic droplets.

In another embodiment, the method further comprises the step of incubating the microfluidic droplets of said first fluid under condition suitable for the growth of said at least one single cell.

In one embodiment, the method according to the first aspect of the present invention further comprises one or more incubation steps carried out before step (b) and/or before step (c) of the method.

Incubation may be performed in any way possible. It is, however, important that a majority of the microfluidic droplets remain intact during the incubation. Independently from where the droplets and microorganisms are incubated, it is preferred that the microorganisms are incubated at appropriate temperatures. The suitable temperature is dependent on the microorganisms in the droplets.

The temperature may vary during incubation or may be constant. In one embodiment of the invention the microfluidic droplets comprising the microorganisms are incubated at a constant temperature. In an alternative embodiment, the microfluidic droplets comprising the microorganisms are incubated at variable temperatures. The incubation time needs to be long enough to allow for the microorganisms to grow. The time is dependent on the medium, the temperature and the microorganisms.

In one embodiment, the temperature during the incubation ranges from 0°C to 100°C, preferably from 4°C to 40°C.

In another embodiment, the incubation time ranges from 1s to 2 months, preferably from 1h to 2 weeks.

The atmosphere may vary during incubation or may be constant. In one embodiment of the invention the microfluidic droplets comprising the microorganisms are incubated at a aerobic atmospheric conditions. In an alternative embodiment, the microfluidic droplets comprising the microorganisms are incubated at microaerophilic conditions. In yet another alternative embodiment, the microfluidic droplets comprising the microorganisms are incubated at anoxic conditions.

In one embodiment, the single-layer microfluidic droplets may optionally comprise a sensing compound which enables the detection and dispensation only of the droplets containing viable microorganism single strain.

When the sensing compound is absent in the single-layer microfluidic droplet, the detection of the viability of the single strain may be carried out by image-based detection, which selects for single-layer microfluidic droplets comprising viable microorganism single strain.

The dispensation of these microfluidic droplets into a plurality of microtiter plate wells, each comprising an appropriate culture medium, ensures the growth of the single strains in a well (i.e., monoclonal microbial cultures). In contrast, standard microbiology methods often require extensive purification steps, which are time consuming. Also, these additional steps might increase the probability of contamination of the sample.

In one embodiment, the microorganism sample comprises microorganisms selected from the group consisting of bacteria, archaea, viruses and fungi.

In the context of the present invention, the sample may be obtained from animals, including humans, therefore encompassing biological fluids, solids, tissues and gases. The sample may also encompass environmental sample, such as surface material, water, soil and industrial samples.

In one embodiment, the sensing compound is capable of reacting to a chemical, biological or physical signal produced by the single cell. As used herein, the expression "activated state of a sensing compound" refers to a sensing compound which upon reacting with a chemical, biological or physical signal produced by the single cell generates a detectable signal.

As used herein, the expression "chemical, biological or physical signal" refers to the method of assaying the viability of a single cell. Therefore, for example, a "physical signal" may refer to a growth assay. A "biological signal" may refer to an enzyme assay. A "chemical signal" may refer to an assay capable of assessing the presence of a specific product, e.g., the detection of carbon-based products produced by the microorganism.

An important characteristic of the present invention is that the viability of the single cell is assayed without breakage of the droplets. The required reagents are introduced into the microfluidic droplets in any possible way, for example, by means of picoinjection or fusion with another droplet comprising such reagents.

In another embodiment, the sensing compound is selected from the group consisting of fluorescent dyes, taggants and pigments.

Non-limiting examples of sensing compounds are FM4-64, FM1-43, CFDA, resazurin.

In case of single-layer microfluidic droplets, wherein each droplet comprises a unique culture medium, the method disclosed herein is also conceived to introduce in each of said single-layer droplet different sensing compounds, as the case may require. The concentration of each sensing compound may also vary.

In a second aspect, the present invention relates to a system or kit comprising:
a. a first microfluidic chip for producing a plurality of a single-layer microfluidic droplets, wherein at least one single-layer microfluidic droplet comprises at least one single cell,
b. a first microfluidic device for collecting said plurality of single-layer microfluidic droplets,
c. a second device for adding a sensing compound into said at least one single-layer microfluidic droplet comprising at least one single cell,
d. a second microfluidic chip for producing a double-layer microfluidic droplet, and
e. a dispensing unit.

In the context of the present invention, the term "device" refers to any kind of devices external to the first and/or second microfluidic chip for carrying out processes such as collection of the single-layer microfluidic droplets or injection of the sensing compound into the single-layer microfluidic droplet comprising at least one single cell.

The dispensing unit is intented to distribute the single-layer or double-layer microfluidic droplets comprising a viable single cell into appropriate wells in a microtiter plate.

The dispensing unit may be connected to a software unit capable to monitor the growth of the single strain.

In one embodiment, the dispensing unit is optionally connected to a sensing unit.

As used herein, the term "sensing unit" refers to any devices capable of sensing a chemical, biological or physical signal emitted from the microfluidic droplet. A non-limiting example of a sensing unit is a fluorescence-based flow cytometry device. As used herein, the term "dispensing unit" refers to any devices capable of discriminating the microfluidic droplets based on the emitted signal. A non-limiting example of a dispensing unit is a fluorescence-activated cell sorter (FACS).

In a third aspect, the present invention relates to the use of the method according to the first aspect of the present invention in a system according to the second aspect of the present invention.

### Examples

### Experimental steps to build a cell bank

### Procedure

### Sample preparation

Bacterial fraction was extracted from a natural sample was prepared using a standard protocol (Janssen et al. 2002, Appl Environ Microbiol. 68(5):2391-2396).

### Preparation of microfluidic chips

The microfluidic devices are fabricated using standard procedure of soft lithography in poly(dimethylsiloxane) (PDMS) (Baret et al. 2009, Lab Chip 9(13):1850-1858). 10:1 mixture of PDMS polymer and cross-linker was prepared and poured on a SU-8 mold. The PDMS mixture was then degassed to remove air bubbles and solidified at 70°C for 4h. The solidified PDMS cast was peeled off the mold. The inlet ports and outlet ports of the PDMS cast were added with a 0.75-mm inner-diameter puncher. The PDMS cast was then bonded to a glass slide after both were first treated with oxygen plasma. If necessary, electrodes were included in the picoinjection chip. The picoinjection chip was heated to 90°C then the electrode channel was filled with melted low-temperature solder (51In/32.5Bi/16.5Sn, Indium Corporation).

The single-layer drop-making microfluidic chip, the droplet library chip, and the picoinjection chip were rendered hydrophobic by 1% (v/v) Trichloro(1*H*,1*H*,2*H*,2*H*-perfluorooctyl)silane in HFE7500. The double-layer droplet making chip was rendered hydrophilic by oxygen plasma before the double-layer droplet-making experiment (Kim et al. 2015, Lab Chip 15(15):3163-3169).

### Single-layer droplet preparation using a single microbial growth medium

Single-layer droplets were produced using a prepared single-layer droplet making microfluidic chip and applying standard workflows (Baret et al. 2009, Lab Chip 9(13):1850-1858). The prepared microbiological sample was diluted to ^{∼}0.1 cell per droplet in a specific medium and used as aqueous phase. 1 uM of sulforhodamine was added to the aqueous phase as the droplet marker. 2.5% (w/w) PEG-PFPE amphiphilic block copolymer surfactant in HFE7500 was used as the oil phase. Both of the solutions were loaded into syringes. The syringes were mounted onto Harvard Apparatus pumps. ^{∼}13 pL single-layer droplets were prepared when the oil phase was set to 850 µL/h and the aqueous phase was set to 300µL/h. The droplets were collected in a droplet collection vial pre-filled with 2.5% (w/w) surfactant in HFE7500.

### Single-layer droplet preparation using several microbial growth media

A prepared droplet library chip was used to produce single-layer droplets from several culture media at the same time. The prepared microbiome sample was diluted to around 0.1 cell per droplet in the desired growth media and used as aqueous phase. 100uL of each of the cell suspension in different media was loaded into micropipette tips. These tips were inserted into the aqueous inlet of the droplet library chip. 5% PEG-PFPE amphiphilic block copolymer surfactant in HFE7500 was used as oil phase. A Fluigent pump was connected to the outlet through a droplet collection vial to provide back pressure. The back pressure was set to -300 mbar. ^{∼}13 pL single-layer droplets from 54 different culture media were prepared at the same time and collected in a droplet collection vial pre-filled with 5% (w/w) surfactant in HFE7500.

After collection, optionally, the droplets were incubated at 30°C for 3-7 days.

### Assay of bacteria growth in droplets

If necessary, a fluorescence dye was picoinjected into each droplets to assay the bacterial growth inside the droplets (Abate et al. 2010, PNAS 107(45):19163-19166). In this experiment, CFDA was used as viability marker. The picoinjection was done as follows.

10 µg/mL of CFDA solution was prepared in the microbiological culture medium. The incubated droplets contained in a specific droplet collection vial were taken out the incubator prior to the picoinjection experiment.

2.5% (w/w) PEG-PFPE amphiphilic block copolymer surfactant in HFE7500 was used as oil phase. The oil and the CFDA solution were loaded separate syringes. The droplet collection vial with single-layer droplets was connected to a syringe filled with 2.5% (w/w) surfactant in HFE7500. After connecting all the components to the picoinjection chip, the flowrate of the oil phase was set to 750 µL/h, of the droplets to 500 µL/h, and of the CFDA solution to 50 µL/h during picoinjection. A 30 kV, 30 kHz voltage was added to the chip during the picoinjection.

After picoinjection, the droplets were collected in the droplet collection vial filled with 2.5% (w/w) surfactant in HFE7500.

### Preparation of double-layer droplets

Double-layer droplets were prepared according to a standard workflow (Zinchenko et al. 2014, Anal. Chem. 86(5):2526-2533). The droplet collection vial with single-layer droplets was connected to a syringe filled with 2.5% (w/w) surfactant in HFE7500. 4% (w/w) Tween20 and 1% (w/v) PluronicF68 in 40 mM NaCI solution was used as aqueous phase. During the preparation of double-layer droplets, the flow rate for the droplets was set ag 500 µL/h and for the aqueous phase at 2500 µL/h. The double-layer droplets were collected in a microcentrifuge tube.

### FACS dispensation

A fluorescence-activated cell sorter (FACS) Aria III (BD) was used to sort the double-layer droplets to select and recover droplets with bacterial growth. 30 uL of the double-layer droplets were added to 3 mL of 2% Pluronic F-68 in 40mM NaCl solution. The sample was loaded to FACS. 488 nm and 561 nm were used to detect the signal from CFDA and sulforhodamine, respectively. The double-layer droplets with high CFDA fluorescence intensity were selected and dispensed into 96- or 384-well microtiter plates whose wells were pre-filled with 80 µL of the culture medium.

After the FACS dispensation, the microtiter plates were incubated at 30°C for 7 days to allow bacterial growth.

### Identification of the strains after dispensation

The regrowing strains after dispensation and incubation were identified using sequencing the full length of the 16S rRNA gene.

## Claims

1. A method of producing a library of microorganisms, the method comprising the steps of:
a. providing a first fluid comprising at least one single cell,
b. dispersing said first fluid comprising at least one single cell in a second fluid, thereby obtaining a plurality of single-layer microfluidic droplets, wherein at least one single-layer microfluidic droplet comprises at least one single cell, wherein the second fluid is immiscible with the first fluid,
c. optionally, adding to said at least one single-layer microfluidic droplet a third fluid comprising a sensing compound, wherein the third fluid is miscible with said first fluid, and wherein the third fluid is immiscible with said second fluid,
d. injecting said at least one single-layer microfluidic droplet optionally comprising the sensing compound into a fourth fluid, wherein said fourth fluid is immiscible with said second fluid, thereby obtaining at least one double-layer microfluidic droplet,
e. dispensing said at least one double-layer microfluidic droplet into a culture medium based on the viability of the cell,
f. incubating said culture medium, thereby obtaining said library.

2. The method according to claim 1, wherein the first fluid, the third fluid and the fourth fluid are hydrophilic and the second fluid is hydrophobic or *vice versa.*

3. The method according to the claims 1 and 2, wherein the single-layer microfluidic droplet is a water-in-oil emulsion droplet or *vice versa.*

4. The method according to any of the claims 1 to 3, wherein the single cell is obtained by diluting a microorganism sample comprising a plurality of strains.

5. The method according to claim 4, wherein the microorganism sample comprises microorganisms selected from the group consisting of bacteria, archaea, viruses and fungi.

6. The method according to any of the claims 1 to 5, wherein the sensing compound is capable of reacting to a chemical, biological or physical signal produced by the single cell.

7. The method according to any of the claims 1 to 6, wherein the sensing compound is selected from the group consisting of fluorescent dyes, taggants and pigments.

8. The method according to any of the claims 1 to 7, wherein each of the plurality of single-layer microfluidic droplets comprise an identical culture medium.

9. The method according to any of the claims 1 to 7, wherein each of the plurality of single-layer microfluidic droplets comprise a unique culture medium.

10. A system comprising
a. a first microfluidic chip for producing a plurality of single-layer microfluidic droplets wherein at least one single-layer microfluidic droplet comprises at least one single cell,
b. a first microfluidic device for collecting said plurality of single-layer microfluidic droplets,
c. a second microfluidic device for adding a sensing compound into said at least one single-layer microfluidic droplet comprising at least one single cell,
d. a second microfluidic chip for producing a double-layer microfluidic droplet, and
e. a dispensing unit.

11. Use of the method according to any of the claims 1 to 9 in a system according to claim 10.
